# EUROPÄISCHE PATENTANMELDUNG

(11) **EP 0 576 860 A2**
(43) Veröffentlichungstag der Anmeldung: **05.01.1994**
(21) Anmeldenummer: 93108999.9
(22) Anmeldetag: 04.06.1993
(51) Int. Cl.: C12P 7/24

(54) **Verfahren zur Herstellung von Coniferylaldehyd**

(30) Priorität: 17.06.1992 DE 4219770
(71) Anmelder: HAARMANN & REIMER GMBH, D-37601 Holzminden (DE)
(72) Erfinder: Abraham, Wolf-Rainer, Dr., D-3171 Hillerse (DE); Arfmann, Hans-Adolf, Dr., D-3300 Braunschweig (DE)
(74) Vertreter: Petrovicki, Wolfgang, Dr.

(57) **Zusammenfassung**

Aus Eugenol läßt sich mit Hilfe von Fungi oder deren Inhaltsstoffen Coniferylaldehyd herstellen.

## Beschreibung

Die Erfindung betrifft ein Verfahren zur Herstellung von Coniferylaldehyd aus Eugenol mit Hilfe von Mikroorganismen oder deren Inhaltsstoffen: 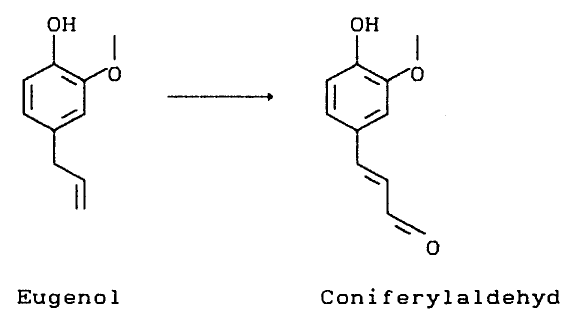
Coniferylaldehyd besitzt interessante organoleptische Eigenschaften. Außerdem ist er bislang nicht in wirtschaftlichem Umfang zugänglich gewesen. In der DE-OS 3 604 874 wird ein Verfahren zur Herstellung von Coniferylaldehyd aus Eugenol mit Hilfe von Mutanten von Arthrobacter globiformis DSM 3597 beschrieben Die entsprechenden Mutanten selbst sind weder hinterlegt worden noch enthält die DE-OS 3 604 874 eine Beschreibung eines reproduzierbaren Verfahrens zu ihrer Gewinnung. Es fehlt also eine ausreichende Offenbarung.

Es wurde nun gefunden, daß sich Coniferylaldehyd aus Eugenol in Gegenwart von Fungi oder ihren Inhaltsstoffen in guten Ausbeuten herstellen läßt.

Gegenstand der Erfindung ist also ein Verfahren zur Herstellung von Coniferylaldehyd aus Eugenol, wonach man Eugenol mit Fungi oder ihren Inhaltsstoffen zusammenbringt, reagieren läßt und den gebildeten Coniferylaldehyd abtrennt.

Bevorzugte Fungi umfassen Ascomyceten, Fungi imperfecti und Phycomyceten.

Zu den bevorzugten Ascomyceten gehören die Gattungen Gibberella (beispielsweise G. baccata, G. cyanea, G. fujikuroi, G. sanbinetti und G. zeae, z.B. IFO 5269), Chaetomium (beispielsweise Ch. cochlioides wie ATCC 10 195, Ch. funicolum, Ch. globosum, Ch. species und Ch. succineum), Cochliobolus (beispielsweise C. miyabeanus), Glomerella (beispielsweise G. cingulata, G. fluctigena, G. fusaroides, G. glycines, G. gossypii, G. lagenarium, G. major, G. mume, G. phacidiomorpha und G. rubicola).

Zu den bevorzugten Fungi imperfecti gehören die Gattungen Aspergillus (beispielsweise A. aculeatus, A. alliaceus, A. amstelodami, A. asperescens, A. astianus, A. atropurpureus, A. aureus, A. auricomus, A. avenaceus, A. awamori, A. batatae, A. brevipes, A. butyracea, A. caesiellus, A. caespitosus, A. candidus. A. carbonarius, A. carneus, A. chevalieri, A. cinnamomeus, A. citri, A. citrisporus, A. clavatus, A. conicus, A. echinulatus, A. effusus, A. elegans, A. fischeri, A. flavipes, A. flavus, A. fonsecaeus, A. fumigatus, A. giganteus, A. glaucus, A. gracilis, A. gymnosardae, A. herbariorum, A. inuii, A. itaconicus, A. janus, A. japonicus, A. kanagawaensis, A. kawachii, A. luchuensis, A. mangini, A. melleus, A. minimus, A. miyakoensis, A. mollis, A. montevidensis, A. nakazawai, A. nidulans, A. niger, z.B. ATCC 9142, A. niveo-glaucus, A. niveus, A. ochraceus, A. oryzae, A. ostianus, A. panamensis, A. parasiticus, A. penicilloides, A. phoenicis, A. proliferans, A. pseudoglaucus, A. pulverulentus, A. quadrilineatus, A. rehmii, A. repens, A. restrictus, A, ruber, A. rugulosus, A. saitoi, A. sclerotiorum, A. sojae, A. sparsus, A. species, A. sulphureus, A. sydowi, A. tamarii, A. terreus, A. terricola, A. unguis, A. usamii, A. ustus, A. varians, A. variecolor, A. versicolor, A. violaceofuscus und A. wentii), Beauveria (beispielsweise B. bassiana und B. species), Botryodiplodia (beispielsweise B. theobromae), Cercospora (beispielsweise C. apii, C. baticola, C. calotropidis, C. canescens, C. chenopodii, C. cladosporioides, C. cruenta, C. diazu, C. fusca, C. hibsci-cannabini, C. italica, C. kaki, C. kikuchii, C. lagenarum, C. macrospora, C. malvacearum, C. medicaginis, C. melongenae, C. melonis, C. musae, C. musarum, C. nicotianae, C. oryzae, C. rosicola, C. scirpicola, C. sesami, C. taiwanensis, C. vaginae, C. violae, C. zebrina und C. zinniae), Corynespora (beispielsweise C. casaiicoli, z.B. DSM 62 474), Curvularia (beispielsweise C. brachyspora, C. falcata, C. fallax, C. geniculata, C. inaequalis, C. lunata, C. maculans, C. oryzae, C. pallescens, z.B. DSM 62 482, C. species, C. tetramera, C. trifolii und C. uncinata), Gliocladium (beispielsweise G. catenulatum, G. deliquescens, G. luteolum, G. roseum und G. species), Diplodia (beispielsweise D. gossypina, z.B. ATCC 10 936, D. natalensis und D. tubericola), Fusarium (beispielsweise F. aquaeductum, F. arthoceras, F. aspidioti, F. avenaceum, F. batatatis, F. bulbigenum, F. buxicola, F. caucasicum, F. cocophilum, F. coeruleum, F. concolor, F. culmorum, F. dimerum, F. diversisporum, F. equiseti, F. expansum, F. fujikuroi, z.B. DSM 893, ATCC 12 764 und ATCC 14 842, F. gibosum, F. gigas, F. graminearum, z.B. DSM 62 722, F. herberum, F. heterosporum, F. inflexum, z.B. DSM 63 203, F. javanicum, F. lateritium, F. lini, F. lycopersici, F. macroceras, F. merismoides, F. microcrea, F. moniliforme, z.B. DSM 840, DSM 764 und DSM 768, F. nivale, F. niveum, F. niveus, F. orthoceras, F. oxysporum, z.B. ATCC 7601, F. oxysporum f. sp. achmeae, z.B. DSM 62 297, oxysporum f. sp. pisi, z.B. ATCC 9991, F. poae, F. pruni, F. redolens, F. reticulatum, z.B. DSM 62 719, F. roseum, z.B. DSM 3019, F. sambucinum, F. sarcochroum, F. semitectum, F. solani, z.B. DSM 62 413, DSM 1164 und DSM 62 416, F. species, F. sphaeriae, F. sporotrichella, F. sporotrichioides, F. sulphureum, F. tabacinum, z.B. DSM 2125, F. udum und F. vasinfectum), Penicillium (beispielsweise P. aculeatum, P. adametzi, P. albidum, P. asperum, P. atramentosum, P. arvense, z.B. CBS 513.74, P. aurantio-violaceum, P. aurantio-virens, P. avellaneum, P. biforme, P. brefeldianum, P. brevicompactum, P. camemberti, z.B. ATCC 4845, P. canescens, P. casei, P. caseicolum, P. charlesii, P. chermesinum, P. chrysogenum, P. citreo-viride, P. citrinum, P. claviforme, P. clavigerum, P. commune, P. corylophilum, P. corymbiferum, P. crustosum, P. cyaneofulvum, P. cyaneum, P. cyclopium, P. daleae, P. decumbens, P. digitatum, z.B. DSM 62 840, P. diversum, z.B. CBS 320.48, P. duponti, P. egyptaceum, P. ehrlichii, P. expansum, P. fellutanum, P. frequentans, P. funiculosum, P. fuscum, P. gladioli, P. godlewskii, P. granulatum, P. helicum, P. herquei, P. humuli, P. implicatum, P. islandicum, P. italicum, P. janthinellum, P. javanicum, P. jensenii, P. kapuscinskii, P. lanoso-coeruleum, P. lanoso-griseum, P. lanoso-viridi, P. lanesum, P. lavendulum, P. levitum, P. lilacinum, P. lividum, P. luteum, P. martensii, P. melearginum, P. melinii, P. miczynskii, P. multicolor, P. nalgiovensis, P. namyslowskii, P. nigricans, P. notatum, P. novae-zeelandii, P. ochraceum, P. ochro-chlorum, P. olivino-viride, P. oxalicum, P. palitans, P. pallidum, P. parvum, P. patulum, P. phoenicum, P. piscarium, P. psittacinum, P. puberulum, P. pulvillorum, P. purpurescens, P. purpurogenum, P. pusillum, P. raciborskii, P. raistrickii, P. restrictum, P. restriculosum, P. rolfsii, P. roqueforti, P. roseo-purpureum, P. rubrum, P. rugulosum, P. sclerotiorum, P. simplicissimum, P. solitum, P. soppi, P. species, P. spiculisporum, P. spinulosum, P. steckii, P. stoloniferum, P. striatum, P. subalteritium, P. tardum, P. terlikowski, P. terrestre, P. thomii, P. trzebinskii, P. turbatum, P. urticae, P. variabile, P. vermiculatum, P. verruculosum, z.B. ATCC 10 483, P. vinaceum, P. viridicatum, P. waksmanni und P. wortmanni) und Stachybotrys (beispielsweise St. species).

Zu den bevorzugten Phycomyceten gehören die Gattungen Absidia (beispielsweise A. coerulea, z.B. ATCC 8990, A. cylindrospora, A. glauca, A. hyalospora, A. orchidis, A. ramosa, A. regnieri, A. repens und A. species), Cunninghamella (beispielsweise C. africana, C. albidia, C. bainieri, C. blakesleeana, C. echinulata, C. elegans, C. homothallica, C. ramosa, C. species und C. verticillata), Mortierella (beispielsweise M. alpina, M. bainieri, M. candelabrum, M. isabellina, M. marburgensis, M. oligospora, M. polycephala, M. pusilla, M. tuberosa und M. zonata), Mucor (beispielsweise M. adriaticus, M. adventitius, M. angulisporus, M. berolinensis, M. buntingii, M. christianensis, M. circinelloides, M. circinelloides f. lusitanicus, z.B. CBS 277.48, M. corymbifer, M. dimorphosporus, M. dispersus, M. dubius, M. erectus, M. genevensis, M. globosus, M. glomerula, M. griseocyanus, M. guilliermondii, M. hiemalis, M. humicola, M. humilis, M. hypochninus, M. javanicus, M. mandshuricus, M. microsporus, M. mucedo, M. murorum, M. parasiticus, M. piriformis, M. plumbeus, M. pusillus, M. racemosus, M. ramannianus, M. rouxianus, M. rouxii, M. simplex, M. solani, M. species, M. sphaerospora, M. spinosus, M. stolonifer, M. varians und M. vuillemini) sowie Rhizopus (beispielsweise Rh. arrhizus, Rh. cambodjae, Rh. chinensis, Rh. chiuniang, Rh. cohnii, Rh. delemar, Rh. formosensis, Rh. japonicus, Rh. javanicus, Rh. kansho, Rh. kasanensis, Rh. nigricans, Rh. niveus, Rh. nodosus, Rh. oryzae, Rh. pseudochinensis, Rh. pygmaeus, Rh. reflexus, Rh. shangaiensis, Rh. species, Rh. stolonifer, Rh. suinus, Rh. tonkinensis und Rh. tritici).

Die für das erfindungsgemäße Verfahren bevorzugtesten Fungi sind die der Gattung Fusarium; die bevorzugtesten Arten sind Fusarium moniliforme, wie z.B. DSM 840 und DSM 768, und Fusarium fujikuroi, wie z.B. F. f. DSM 893. Ebenso bevorzugt ist die synonyme Gibberella fujikuroi (s. J.C. Corell, Phytopathology 81 (1991), 1061-1064), z.B. ATCC 12 764 und ATCC 14 842.

Das erfindungsgemäße Verfahren kann so ausgeführt werden, daß man Fungi in Gegenwart von Eugenol fermentiert und den erhaltenen Coniferylaldehyd isoliert. Statt der Fungi kann man auch ihre Inhaltsstoffe, d.h. ihre Zellflüssigkeit oder die darin enthaltenen Enzyme einsetzen.

Wird das erfindungsgemäße Verfahren durch Fermentieren von Fungi durchgeführt, empfiehlt sich der Zusatz einer C-Quelle. Als solche eignen sich beispielsweise aliphatische C₆-C₁₈-, vorzugsweise C₈-C₁₄-Kohlenwasserstoffe, wie z.B. n-Hexan, n-Octan, n-Dodecan, die Isomeren dieser Verbindungen, die Mischungen der genannten Kohlenwasserstoffe einschließlich technischer Gemische, wie z.B. Kerosin. Die Fermentierung kann in der C-Quelle als Medium stattfinden.

Es hat sich als vorteilhaft erwiesen, die Kultur von der Eugenol-Zugabe mit einem Induktor zu versetzen. Als Induktoren sind beispielsweise wirksam:
C₂-C₂₀-Carbonsäure-C₁-C₁₂-alkylester wie Essigsäurebutylester und Ölsäurepropylester, substituierte Benzole wie Propylbenzol, Allylbenzol, n-Butylbenzol, 1,4-Diethylbenzol, substituierte Phenole, unsubstituierte und substituierte Phenolether, wie z.B. 2-Allylphenol, 2-Allyl-6-methyl-phenol, 4-Methoxy-3-hydroxystyrol, Eugenol, Isoeugenol, Eugenol- und Isoeugenolmethylether, Anisol, 4-Vinyl- und 4-Allylanisol, Phenetol, Veratrol, Veratrylalkohol, Anethol, Carvacrol, Safrol, Isosafrol, Vanillylalkohol, C₂-C₁₈-Carbonsäure-eugenol- und -isoeugenolester wie z.B. Eugenol- und -isoeugenolacetat, -butyrat und -oleat.

Das Ausgangsprodukt Eugenol setzt man in der Regel in einer Menge von 0,1 bis 20, vorzugsweise von 0,3 bis 3 g, bezogen auf 1 l Kulturbrühe, ein. Es kann pur oder in Form einer Lösung zugesetzt werden. Zur Herstellung der Eugenollösung werden wasserverdünnbare organische Lösungsmittel wie beispielsweise Ethanol oder Dimethylformamid bevorzugt.

Die C-Quelle setzt man im allgemeinen in einer Menge von 0,2 bis 200, vorzugsweise 0,5 bis 20 g, bezogen auf 1 l Kulturbrühe, ein.

Der Induktor wird im allgemeinen in einer Menge von 5 bis 2000, vorzugsweise von 10 bis 500 mg, bezogen auf 1 l Kulturbrühe, eingesetzt. Will man mit Induktor arbeiten, geschieht dies am besten so, daß man das die Fungikultur enthaltende Kulturmedium mit dem Induktor induziert und die Fungi 0,5 bis 3 Tage kultiviert. Dann kann das Eugenol zugegeben werden. Man kann aber auch das Myzel abtrennen und für das erfindungsgemäße Verfahren einsetzen. Schließt man das Myzel vorher auf, trennt die Zelltrümmer ab und setzt das Eugenol dem zellfreien Extrakt zu, so kann es sein, daß man Coniferylalkohol erhält, der dann mit dem Kulturüberstand zu Coniferylaldehyd oxidiert werden kann.

Das Fortschreiten der Reaktion läßt sich dünnschichtchromatografisch verfolgen.

Die Aufarbeitung erfolgt im allgemeinen so, daß man Überstand und Myzel trennt, mit organischem Lösungsmittel (z.B. Ethylacetat) extrahiert, das Lösungsmittel abzieht und den Rückstand reinigt, beispielsweise durch Chromatographie.

### Beispiele

Für die Vorkultur wird zunächst ein 100 ml Erlenmeyerkolben mit 20 ml sterilisiertem Kulturmedium, enthaltend 10 g/l n-Dodecan, 3 g/l Natriumnitrat, 1 g/l Hefeextrakt, 1,3 g/l K₂HPO₄ x 3 H₂O, 0,5 g/l KCl, 0,5 g/l MgSO₄ x 7 H₂O, 0,05 g/l FeSO₄ x 7 H₂O, 0,7 g/l Zitronensäure x 1 H₂O, das vor dem Autoklavieren auf pH 4,5 eingestellt wurde, mit einer ausgewachsenen Schrägagarkultur der geeigneten Mikroorganismen beimpft und 24 bis 72 h bei 27°C und 140 UpM inkubiert. Anschließend wird diese Kultur unter aseptischen Bedingungen in einen 1 000 ml Erlenmeyerkolben mit 200 ml Kulturmedium überführt, gleichzeitig mit einem geeigneten Induktor induziert und 24 bis 48 h unter o. a. Bedingungen kultiviert.

Das Substrat, pur oder gelöst in Dimethylformamid oder Ethanol (50 mg/ml), wird dann in einer Substratkonzentration von 0,5 bis 2 g/l dazugegeben. Der Verlauf der Umwandlung wird dünnschichtchromatografisch verfolgt. Dazu wird 1 ml einer steril entnommenen Probe mit 0,2 ml Ethylacetat versetzt, 2 min geschüttelt und 2 min zentrifugiert. Vom Überstand werden 20 µl auf HPTLC-Fertigplatten, Kieselgel Si-60 (Merck), aufgetragen, mit n-Hexan/Ethylacetat 6:4 (Volumenverhältnis) entwickelt, im UV (254 oder 366 nm) betrachtet und anschließend mit Anisaldehyd-Sprühreagenz (0,6 ml Anisaldehyd, 1,0 ml Schwefelsäure, 50 ml Essigsäure) besprüht und 2 min bei 100°C entwickelt. Die Oxidationsprodukte werden dadurch als farbige Flecke sichtbar.

Für die präparative Aufarbeitung werden Überstand und Myzel getrennt, jeweils dreimal mit Ethylacetat extrahiert, die Extrakte vereinigt und das Lösungsmittel im Vakuum vertrieben.

Die chromatografische Trennung erfolgt an Kieselgel (Si 60, Korngröße 40 bis 63 µm, Merck) mit n-Hexan/Ethylacetat im Gradientenbetrieb. Das Startverhältnis ist 100:0 n-Hexan/Ethylacetat und das Endverhältnis liegt bei 6:4 (Verhältnisse bezogen auf Volumenanteile).

### Beispiel 1

Diplodia gossypina ATCC 10936 wird in 800 ml des o. g. Mediums mit n-Dodecan als C-Quelle angeimpft und mit 400 mg Eugenololeat beim Animpfen induziert. Nach 3 Tagen Wachstum werden 400 mg Eugenol dazugegeben. Nach 143 h Kontaktzeit werden 45 mg Coniferylaldehyd (10,4 % d. Th.) neben 100 mg unumgewandeltem Eugenol isoliert.

### Beispiel 2

Verfährt man wie in Beispiel 1, setzt aber statt Eugenololeat 25 ppm Eugenolacetat als Induktor ein und verwendet den Pilz Fusarium moniliforme DSM 764, erhält man 252 mg Coniferylaldehyd (58 % d. Th.).

### Beispiel 3

Verfährt man wie in Beispiel 1, setzt aber Fusarium moniliforme DSM 840 und Eugenolmethylether in einer Konzentration von 25 ppm ein, induziert 24 h lang, und erhöht die Substrat-Konzentration auf 2 g/l, erhält man nach 72 h 999 mg Coniferylaldehyd (57,5 % d. Th.).

### Beispiel 4

Verfährt man wie in Beispiel 3, zentrifugiert aber nach der Induktion das Myzel ab, suspendiert es in einem Phosphat-Puffer (pH 7,0) und setzt dazu 2 g Eugenol ein, erhält man nach 143 h 991 mg Coniferylaldehyd (45 % d. Th., 57,8 % d. Umw.) neben 420 mg Eugenol.

### Beispiel 5

Verfährt man wie in Beispiel 4, schließt aber das Myzel auf, trennt die Zelltrümmer ab und setzt dem zellfreien Extrakt 2 g Eugenol zu, erhält man nach 24 h 910 mg Coniferylalkohol. Dieser kann mit dem Kulturüberstand quantitativ zum Coniferylaldehyd oxidiert werden.

## Patentansprüche

1. Verfahren zur Herstellung von Coniferylaldehyd aus Eugenol, wonach man Eugenol mit Fungi oder ihren Inhaltsstoffen zusammenbringt, reagieren läßt und den gebildeten Coniferylaldehyd abtrennt.

2. Verfahren nach Anspruch 1, wonach man als Fungi Ascomyceten, Fungi imperfecti oder Phycomyceten einsetzt.

3. Verfahren nach Anspruch 1, wonach man Fungi der Gattungen Absidia, Aspergillus, Chaetomium, Corynespora, Curvularia, Diplodia, Gibberella, Fusarium, Mucor oder Penicillium verwendet.

4. Verfahren nach Anspruch 1, wonach man Fungi aus der Reihe bestehend aus Absidia coerulea, Aspergillus niger, Corynespora cassiicola, Curvularia pallescens, Chaetomium cochlioides, Diplodia gossypina, Fusarium graminearum, Fusarium fujikuroi, Fusarium inflexum, Fusarium moniliforme, Fusarium oxysporum, Fusarium oxysproum f. aechmeae, Fusarium oxysporum f. sp. pisi, Fusarium reticulatum, Fusarium roseum, Fusarium solani, Fusarium tabacinum, Gibberella zeae, Mucor circinelloides f. lusitanicus, Penicillium arvense, Penicillium camemberti, Penicillium digitatum, Penicillium diversum und Penicillium verruculosum verwendet.

5. Verfahren nach Anspruch 1, wonach man die Kultur vor der Eugenolzugabe mit einem Induktor versetzt.

6. Verfahren nach Anspruch 5, wonach man als Induktoren Eugenol, Eugenolmethylether, Eugenolester (wie Eugenolacetat, Eugenololeat), Safrol, Isoeugenol, Isoeugenolacetat, Vanillylalkohol, n-Butylbenzol oder 4-Allylanisol einsetzt.
